# FASCICULE DE BREVET EUROPEEN

(11) **EP 2 498 744 B1**
(45) Date de publication et mention de la délivrance du brevet: **08.08.2018**
(21) Numéro de dépôt: 10795421.6
(22) Date de dépôt: 05.11.2010
(51) Int. Cl.: A61K 8/06, A61K 8/894, A61Q 3/02, A61K 8/39

(54) **PROCEDE D'INCORPORATION D'ACTIFS DANS UN VERNIS A ONGLES ET VERNIS A ONGLES AINSI PREPARE**
VERFAHREN ZU EINFÜHRUNG EINES WIRKSTOFFES IN EINEM NAGELLACK UND DER DAMIT ERHALTENEN NAGELLACK
METHOD FOR INCORPORATING ACTIVE AGENTS IN A NAIL COATING AND THE OBTAINED ENAMAIL

(30) Priorité: 10.11.2009 FR 0905388
(43) Date de publication de la demande: 19.09.2012
(73) Titulaire: Fiabila, 28130 Maintenon (FR)
(72) Inventeur: BONNEVIE, Xavier, F-28300 Mainvilliers (FR)
(74) Mandataire: Le Cloirec, Claudine
(86) Numéro de dépôt international: PCT/FR2010/052384
(87) Numéro de publication internationale: WO 2011/058266

(56) Documents cités:
- WO-A1-96/41612
- FR-A1- 2 819 401
- US-A- 5 102 654
- US-A- 5 484 586
- US-A1- 2008 139 453

## Description

La présente invention concerne le domaine des vernis à ongles, et plus particulièrement un procédé d'incorporation d'agents actifs dans des compositions de vernis à ongles.

De manière classique, les compositions de vernis à ongles sont à base de solvant(s). Ils renferment généralement :
- un agent filmogène, habituellement de la nitrocellulose ou un dérivé nitrocellulosique,
- un plastifiant,
- parfois un agent thixotropant,
- au moins une résine, et
- un ou plusieurs solvants,
- des colorants et/ou des pigments.

Plus récemment ont été mis au point des vernis en phase aqueuse. Ils renferment :
- un agent filmogène, tel qu'un liant acrylique ou styrène acrylique,
- un ou des coalescents,
- un ou des plastifiants,
- de l'eau,
- des colorants et/ou des pigments,
- des agents épaississants et thixotropants.

Pour des applications particulières, par exemple pour le renforcement de l'ongle ou pour son hydratation, ces vernis peuvent comporter sous forme d'additifs, des agents actifs sur l'ongle, tels que des agents hydratants et des agents durcisseurs de l'ongle.

Ces composés peuvent être dégradés au cours du stockage de la composition ou lors de son application sur l'ongle. Certains de ces additifs peuvent également déstabiliser le vernis et réduire ses propriétés, telles que ses propriétés de dureté, de brillance...etc., même s'ils sont introduits en faibles quantités. La plupart de ces composés ne peuvent être incorporés qu'en petite quantité dans la composition de vernis, le plus souvent en raison soit de leur incompatibilité avec le solvant du vernis, soit de leur très faible solubilité dans ce dernier, ce qui conduit au fait qu'ils s'intègrent difficilement dans la composition de vernis.

Certains actifs spécifiques ont pu être introduits dans des compositions de vernis avec l'ajout de stabilisants spécifiques à la molécule active : par exemple, dans le brevet EP 0.931.538 l'ajout de N-chlorosuccinimide ou d'acide borique, ou encore dans le brevet US 4,897,261 l'introduction de polymères de poids moléculaire important (> 1000) comportant une partie hydrophile et une partie lipophile.

Le but principal de la présente invention est donc de pallier les inconvénients ci-dessus en proposant un procédé de préparation de composition de vernis à ongles, simple, réalisable à température ambiante, et par incorporation d'agents actifs sur l'ongle, qui permette sans ajout d'acide ni de polymère comme dans les documents de l'Art Antérieur, de protéger ces actifs dans ladite composition de vernis.

A cet effet, l'invention concerne un procédé d'incorporation, dans une composition de vernis à ongles renfermant un agent filmogène, d'un agent actif sur l'ongle, du type sels minéraux et/ou composé organique telle que vitamine, extrait de plantes, huiles essentielles, en vue de la préservation dudit actif, caractérisé en ce qu'il comprend les étapes successives suivantes :
a) préparation d'une émulsion du type huile dans eau ou eau dans huile, par mise en contact, sous agitation, d'au moins une première phase constituée dudit agent actif, ou d'une solution le solubilisant, et d'une seconde phase dans laquelle l'agent est insoluble ou peu soluble, en présence d'au moins un tensio-actif, de manière à obtenir une émulsion simple, stable, constituée de deux phases, la première phase, discontinue, étant présente sous forme de gouttelettes au sein de la seconde phase, continue, ladite seconde phase étant soluble dans la composition de vernis,
b) puis introduction, sous agitation, de ladite émulsion dans une composition de vernis, dans des proportions comprises entre 0,01 % et 5% en poids de la composition de vernis.

Ainsi l'agent actif pour l'ongle est présent dans une phase non miscible avec le solvant dudit vernis et/ou dans une phase distincte de la phase solvant dudit vernis, de manière à être protégé lors du stockage du vernis et libéré au contact de l'ongle lors de l'application du vernis sur l'ongle.

Les proportions pondérales respectives des phases de l'émulsion sont comprises entre 20-80 et 80-20 environ, de préférence entre 35-65 et 65-35.

De manière avantageuse, il est également possible, selon l'invention, de prévoir que le procédé comprenne une étape a)bis dans laquelle l'émulsion simple obtenue à l'issue de l'étape a) est, avant l'étape b) d'introduction dans la composition de vernis, incorporée, sous agitation, dans une troisième phase dans laquelle la seconde phase de l'émulsion simple est insoluble, de manière à obtenir une émulsion multiple, stable, constituée de trois phases, ladite troisième phase étant soluble dans la composition de vernis.

L'émulsion peut avantageusement comprendre une phase aqueuse et une phase "huile" ou phase "solvant". De manière préférée, la phase "solvant" de l'émulsion comprend un ou plusieurs solvants organiques peu ou pas miscibles dans l'eau tels que des acétates d'alkyle en C₂-C₈, par exemple les acétates d'éthyle et/ou de butyle, traditionnellement utilisés dans des compositions de vernis à base de solvant.

Il est important, pour obtenir des émulsions stables, d'ajouter au moins un tensio-actif dans au moins une des phases de l'émulsion. Ce tensio-actif ou ce mélange de tensio-actifs est choisi parmi les tensio-actifs cationiques, anioniques, amphotériques ou non-ioniques, de préférence, de faible masse molaire, inférieure à 1000, avantageusement inférieure à 500 environ. De manière préférée, le tensio-actif non-ionique présente un indice HLB (Hydrophilic Lipophilic Balance) compris entre 0 et 20, avantageusement entre 2 et 8 pour les émulsions de type "eau-dans-huile", et avantageusement entre 12 et 16 pour les émulsions de type "huile-dans-eau".

Ainsi, il est possible d'introduire l'émulsion dans la composition de vernis selon des proportions comprises entre 0,01 et 1 % en poids, en poids de ladite composition de vernis. Ces proportions correspondent par exemple à des teneurs en eau très faibles (à savoir inférieures à 1 % en poids) dans un vernis solvant par rapport à des compositions, telles que celles décrites dans US 5,102,654 (renfermant de 5 à 15 % en poids d'eau) tout en augmentant la teneur en actif dans ledit vernis.

Ceci permet d'introduire l'agent actif pour l'ongle dans des concentrations plus importantes que celles de l'Art Antérieur, permettant ainsi une meilleure action de ce composé à la surface de l'ongle après application de la composition de vernis.

Selon un premier mode de réalisation du procédé de l'invention, lorsque la composition de vernis est une composition à base aqueuse, l'agent actif est présent dans la phase hydrophobe de l'émulsion simple ou dans la phase hydrophile de l'émulsion multiple.

Selon un deuxième mode de réalisation du procédé de l'invention, lorsque la composition de vernis est une composition à base de solvants à caractère plutôt hydrophobe, l'agent actif est présent dans la phase hydrophile de l'émulsion simple ou dans la phase hydrophobe de l'émulsion multiple.

L'agent actif pour l'ongle peut être soit un sel minéral soit une molécule organique. S'il s'agit d'un sel minéral, il peut être choisi parmi les halogénures (de préférence chlorures ou iodures) ou les sulfates, de préférence les sulfates d'aluminium, de cuivre, de magnésium, de manganèse, de fer, de potassium, de calcium et de sodium, ou un mélange de ceux-ci. S'il s'agit d'une molécule organique ou un mélange de molécules organiques, ces dernières peuvent être choisies parmi les vitamines, telles que les vitamines A, B5, C, E, F, H, ou leurs dérivés, des extraits de plantes, de fruits, d'algues, de champignons ou de caviar, des aldéhydes (tels que le citral, l'hexanal...), des huiles végétales (telles que les huiles de soja, ricin, jojoba, noix, olive, mongongo, tournesol...), des huiles essentielles, des acides aminés, des peptides, des protéines, des céramides, l'allantoïne et ses dérivés, des dérivés organiques du silicium, des actifs obtenus par fermentation, des halogénures de benzalkonium et des dialkylesulfones.

Enfin, la présente invention concerne également les compositions de vernis à ongles en tant que telles, renfermant un agent filmogène, obtenues avantageusement par le procédé décrit précédemment dans laquelle au moins un agent actif pour l'ongle est présent dans une phase non miscible avec le solvant dudit vernis et/ou dans une phase distincte de la phase solvant dudit vernis, de manière à être protégé lors du stockage dudit vernis et libéré au contact de l'ongle lors de l'application du vernis sur l'ongle.

Plus particulièrement, selon le premier mode de réalisation, la composition de vernis à ongles étant une composition à base aqueuse, l'agent actif est présent dans une phase hydrophobe ou dans une phase hydrophile en émulsion au sein d'une phase hydrophobe.

Selon le second mode de réalisation la composition de vernis à ongles étant une composition à base de solvants à caractère hydrophobe, l'agent actif est présent dans une phase hydrophile ou dans une phase hydrophobe en émulsion au sein d'une phase hydrophile. Dans ce cas, la phase hydrophile renferme avantageusement une teneur en eau inférieure à 0,5 % en poids.

L'invention va être illustrée ci-après par des exemples non limitatifs.

Dans ces exemples, on cherche à protéger des agents actifs pour l'ongle, dans des compositions classiques de vernis à ongles.

Deux types de vernis ont été mis en oeuvre, des compositions de vernis à base aqueuse (pour lesquelles le solvant majoritaire est l'eau), et des compositions de vernis à base de solvants organiques.

### Exemple 1 : Agents actifs minéraux solubles dans l'eau

Ces agents actifs minéraux sont solubles dans l'eau.

La phase "eau" et la phase "solvant" ont été préparées en mélangeant séparément, sous agitation, les composants suivants :

**Tableau 1**

| Constituants | Parties en poids |
|---|---|
| Phase Eau : | |
| • Eau déminéralisée | 68 |
| • Conservateur | 1 |
| • Mélange d'actifs minéraux solubles dans l'eau | 7 |
| Total | 76 |
| Phase Solvant : | |
| • Nitrocellulose | 3 |
| • Acétate de butyle | 117 |
| • Cetyl PEG/PPG-10/1 Dimethicone (indice HLB=5) | 4 |
| Total | 124 |

Le mélange d'actifs minéraux de la phase "eau" est ici un mélange renfermant :
- 5 parties en poids d'un "multimineral complex II vendu par Procital et contenant des chlorures de Al, Mg, Mn, Na, K, Zn et du sulfate de cuivre et de la lysine,
- et 2 parties d'Atoligomer vendu par Codif qui est un concentré de sels d'eau de mer auquel on a retiré le sodium.

La phase eau est introduite sous agitation dans la phase solvant, puis le mélange est laissé sous agitation 10 minutes à 1500 tours/min.

L'émulsion obtenue est blanche et stable pendant plusieurs mois. Cette émulsion est ensuite introduite sous agitation, dans une proportion de 1 % en poids, dans un vernis nitrocellulosique transparent, dont la composition est détaillée dans le tableau 2 ci-après.

**Tableau2**

| Constituants du vernis | % en poids |
|---|---|
| Acétate d'éthyle | 40 |
| Acétate de butyle | 24 |
| Nitrocellulose (30 % dans alcool isopropylique) | 17 |
| Résine polyester | 11,5 |
| Acétyl tributyl citrate | 7,5 |
| Total | 100 |

Cet exemple montre comment il est possible d'incorporer des sels solubles dans l'eau dans un vernis nitrocellulosique à base de solvant organique, avec une teneur en eau réduite (0,4 % en poids du vernis). Ces oligoéléments sont nécessaires à une bonne croissance de l'ongle. Ils sont libérés sur l'ongle après l'application et le séchage du vernis sur celui-ci.

L'incorporation de l'émulsion eau-dans-solvant dans le vernis du tableau 2 n'en modifie pas la transparence ni les propriétés classiques de ce type de vernis, ni son pH. Le tableau 3 suivant présente les résultats observés. Il n'était pas évident de pouvoir ainsi incorporer une émulsion stable dans une composition liquide, renfermant une fraction importante de solvant.

**Tableau 3**

| propriétés du vernis | renfermant 1 % d'émulsion | sans émulsion |
|---|---|---|
| Dureté | 263 | 210-280 |
| Brillance | 90,7 | 85-92 |
| Adhérence sur verre | 0-1 | 0-1 |
| Temps de séchage | 3 min 15 s | 2 min 30 à 4 min 30 s |
| Teneur en eau | 0,57 % | 0,3 % - 0,5 % |
| Extrait Sec | 27,32 % | 26-28 % |
| Viscosité (6OT/min) | 250 mPa.s | 140-280 mPa.s |

Les caractéristiques physiques du vernis ont été testées selon les méthodes suivantes :
- la brillance est mesurée avec un brillancemètre Minolta 268 (angle d'indice 60°) pour une application faite sur carte type LENETA, et exprimée sur une échelle allant jusqu'à 100 ;
- la dureté est mesurée à l'aide d'un pendule de "Persoz" sur le film sec formé par l'application d'une couche de 100 µm d'épaisseur de vernis sur une plaque de verre, après séchage une nuit à température ambiante (20°C) (les valeurs obtenues sont exprimées en secondes) ;
- l'adhérence sur verre : le film de vernis formé sur verre est griffé en quadrillage avec un peigne de six lames type SHEEN 750/1. Un ruban adhésif appliqué sur les griffures est arraché, et l'on observe que moins de 5 % de la surface du film est arrachée, correspondant à une bonne adhérence, exprimée avec une valeur comprise entre 0 et 1 (sur une échelle de 5) ;
- la viscosité est mesurée au viscosimètre Brookfield LVT avec l'aiguille n° 3 à 25°C à 6 tours/min ou à 60 tours/min pendant une minute, elle permet d'apprécier la bonne aptitude à l'étalement du vernis.

### Exemple 2 : Pantothénate de calcium en solution dans l'eau

Le pantothénate de calcium (dérivé de la vitamine B5) est intéressant pour le renforcement des ongles (ongles cassants) par une action cicatrisante. Cependant, il est très peu soluble dans les solvants organiques habituellement mis en oeuvre dans les vernis à ongles. Or, il s'avère qu'il est très soluble dans l'eau, et peut y être solubilisé à des concentrations importantes.

Le composé est donc dissous dans de l'eau déminéralisée et l'on prépare séparément une phase solvant. Les compositions des deux phases sont les suivantes (tableau 4).

**Tableau 4**

| Constituants | Parties en poids |
|---|---|
| Phase Eau : | |
| . Eau déminéralisée | 61 |
| . Pantothénate de calcium | 15 |
| Total | 76 |
| Phase Solvant : | |
| . Nitrocellulose | 3 |
| . Acétate de butyle | 116 |
| **.** Cetyl PEG/PPG-10/1 Dimethicone (indice HLB=5) | 5 |
| Total | 124 |

La phase eau est introduite sous agitation dans la phase solvant, puis ce mélange est laissé sous agitation 10 minutes à 1500T/min.

L'émulsion eau dans solvant obtenue est blanche et stable à température ambiante (20°C à 25°C environ) pendant plusieurs mois. Cette émulsion simple, à deux phases, est ensuite introduite, sous agitation, selon une proportion de 1 % en poids, dans un vernis nitrocellulosique dont la composition est détaillée dans le tableau 5.

Le vernis est une base thixotrope légèrement pigmentée en blanc :

**Tableau 5**

| Constituants du vernis | % en poids |
|---|---|
| Acétate d'éthyle | 18,7 |
| Acétate de butyle | 38 |
| Nitrocellulose (30 % dans alcool isopropylique IPA) | 18 |
| Résines polyester | 12 |
| Acetyltributylcitrate | 8 |
| Résine styrène acrylique | 2 |
| Stéaralkonium bentonite | 1,3 |
| Oxyde de titane | 2 |
| Total | 100 |

Le pantothénate de calcium a tendance à faire jaunir les vernis nitrocellulosiques ; cependant on a constaté, de manière surprenante, que lorsqu'il est introduit sous forme d'émulsion, cet effet de jaunissement est diminué.

Le tableau 6 ci-après regroupe les propriétés de ce vernis-base incluant 1 % de l'émulsion eau-dans-solvant ci-dessus.

**Tableau 6**

| Propriétés du vernis | renfermant 1 % de l'émulsion | sans émulsion |
|---|---|---|
| Dureté | 172 | 200 |
| Brillance | 88,9 | 88,4 |
| Adhérence sur verre | 0-1 | 0-1 |
| Temps de séchage | 4 min | 3 min 45 s |
| Teneur en eau | 0,60 % | 0,40 % |
| Extrait Sec | 31,09 % | 31,50 % |
| Viscosité (6-60-6 T/min) | 1300/546/880 mPa.s | 2120/670/1200 mPa.s |

Dans cet exemple, l'ajout de l'émulsion fait baisser la dureté (qui est initialement vers 200....). Il a été constaté que le pantothénate de calcium ainsi introduit a une action moins jaunissante sur le vernis, que lorsqu'il est introduit sous la forme d'une solution alcoolique.

L'indice de blancheur (0 = noir et 100 = blanc parfait) a été mesuré par spectrophotométrie de plaques Leneta sur lesquelles a été appliquée une couche de vernis. Les résultats sont les suivants :
Vernis témoin : l'indice de blancheur est de 79,99 à t= 0
Après une semaine à 55°C : l'indice de blancheur est de 77,30 (soit une diminution de 3,36 %)
Vernis avec solution alcoolique de pantothénate de calcium : l'indice de blancheur est de 80,07, puis 68,51 après une semaine (correspondant à -14,43 %)
Vernis avec émulsion de pantothénate de calcium : 79,84 puis 68,89 (-13,71 %)
En parallèle a été réalisé un autre essai avec un autre actif jaunissant (le D-panthénol) qui produit les mêmes résultats : ajouté sous une forme d'émulsion le D-panthénol présente un jaunissement moindre que lorsqu'il est ajouté sous forme d'une solution alcoolique. Ces actifs semblent ainsi protégés de leur dégradation lorsqu'ils sont présents sous forme d'une émulsion.

### Exemple 3 : Vitamine E acétate

Ce composé a été préparé pour former une émulsion du type "huile dans eau", puis introduit sous agitation dans un vernis à l'eau.

La composition des deux phases de l'émulsion est présentée dans le tableau 7.

**Tableau 7**

| Constituants | Parties en poids |
|---|---|
| Phase Huile : | |
| . Vitamine E acétate | 40 |
| . Dérivé d'acide gras modifié non ionique | 10 |
| Total | 50 |
| Phase Eau : . Eau déminéralisée | 49 |
| . Conservateur | 1 |
| Total | 50 |

Le tensio-actif est un dérivé d'acide gras éthoxylé, commercialisé sous le nom Tego Dispers 740 W.

La phase huile est introduite dans la phase eau sous agitation.

L'émulsion obtenue est laiteuse et stable plusieurs mois à température ambiante.

Cette émulsion a ensuite été introduite sous agitation dans un vernis phase aqueuse dans des proportions de 0,5 % en poids du vernis, dont la composition est présentée dans le tableau 8 ci-après.

**Tableau 8**

| Constituants du vernis | % en poids |
|---|---|
| Agent filmogène : Copolymères de styrène/acrylique en émulsion | 76 |
| Coalescent | 4 |
| Plastifiant | 2 |
| Silicate de magnésium et de sodium | 0,7 |
| Conservateur | 0,5 |
| Epaississant organique | 0,5 |
| Agent siliconé de tension et de glissance | 0,5 |
| Pigments | 0,6 |
| Mica titane | 2 |
| Eau déminéralisée | 13,2 |
| Total | 100 |

Dans cet exemple, la forme émulsion permet d'apporter la vitamine E dans un produit en phase aqueuse. En effet, lorsque l'on cherche à l'incorporer directement dans le vernis à l'eau, on constate qu'elle n'est pas du tout miscible et provoque des refus à l'application sur l'ongle comme des mini-gouttes d'huile.

Au contraire, le vernis appliqué avec la vitamine E acétate en émulsion forme une pellicule homogène et régulière sur l'ongle.

### Exemple 4 : émulsion multiple de vitamine E acétate

L'émulsion "huile-dans-eau" de l'exemple 3 est ensuite émulsionnée dans un nouveau milieu solvant renfermant un mélange de tensio-actifs.

L'émulsion obtenue qui devient une émulsion de type "Huile dans Eau dans Solvant" présente la composition suivante (tableau 9).

**Tableau 9**

| Constituants | Parties en poids |
|---|---|
| Phase Solvant : | |
| . Acétate de Butyle | 36 |
| . Acétate d'éthyle | 25 |
| . Nitrocellulose | 10 |
| . Cétyl PEG/PPG - 10/1 Diméthicone (indice HLB = 5) | 0,6 |
| . Sorbitan Sesquioleate (indice HLB=3,7) | 0,4 |
| Total | 72,0 |
| Emulsion Huile dans Eau : | |
| . Emulsion de l'exemple 3 | 28 |

La phase Huile dans Eau introduite dans la phase solvant sous agitation conduit à une émulsion multiple qui est blanche et stable plusieurs mois.

Cette émulsion peut ensuite être introduite sous agitation dans un vernis solvant à une teneur de 1 % en poids dans le vernis présenté dans le tableau 10.

**Tableau 10**

| Constituants du vernis solvant | % en poids |
|---|---|
| Solvants | |
| Acétate de butyle | 40 |
| Acétate d'éthyle | 29,5 |
| Isopropanol | 4,5 |
| Nitrocellulose | 10,5 |
| Acétyl tributyl citrate (ATC) | 6 |
| Résine polyester | 4 |
| Résine acrylique | 1,5 |
| Stearalkonium Hectorite | 1 |
| Pigment | 3 |
| Total | 100 |

Dans tous les exemples cités ci-dessus, les émulsions restent stables lorsqu'elles sont stockées entre 20 et 25°C à l'abri de la lumière.

## Revendications

1. Procédé d'incorporation, dans une composition de vernis à ongles renfermant un agent filmogène, d'un agent actif sur l'ongle, du type sels minéraux et/ou composé organique telle que vitamine, extrait de plantes, huiles essentielles, en vue de la préservation dudit agent actif,
**caractérisé en ce qu'**il comprend les étapes successives suivantes :
a) préparation d'une émulsion du type huile dans eau ou eau dans huile, par mise en contact, sous agitation, d'au moins une première phase constituée dudit agent actif, ou d'une solution le solubilisant, et d'une seconde phase dans laquelle l'agent est insoluble ou peu soluble, en présence d'au moins un tensio-actif, de manière à obtenir une émulsion simple, stable, constituée de deux phases, la première phase, discontinue, étant présente sous forme de gouttelettes au sein de la seconde phase, continue, ladite seconde phase étant soluble dans la composition de vernis,
b) puis introduction, sous agitation, de ladite émulsion dans une composition de vernis dans des proportions comprises entre 0,01 % et 5% en poids de la composition de vernis.

2. Procédé selon la revendication 1, **caractérisé en ce que** l'émulsion est introduite dans la composition de vernis selon des proportions comprises entre 0,1 et 3 % en poids de ladite composition de vernis.

3. Procédé selon la revendication 1 ou 2, **caractérisé en ce qu'**il comprend une étape a)bis dans laquelle l'émulsion simple obtenue à l'issue de l'étape a) est, avant l'étape b) d'introduction dans la composition de vernis, incorporée, sous agitation, dans une troisième phase dans laquelle la seconde phase de l'émulsion simple est insoluble, de manière à obtenir une émulsion multiple, stable, constituée de trois phases, ladite troisième phase étant soluble dans la composition de vernis.

4. Procédé selon l'une quelconque des revendications précédentes, **caractérisé en ce que** le tensio-actif ou le mélange de tensio-actifs, introduit dans au moins l'une des phases de l'émulsion, avant la mise en contact des dites phases, est choisi parmi les tensio-actifs cationiques, anioniques, amphotériques ou non-ioniques.

5. Procédé selon l'une quelconque des revendications précédentes, **caractérisé en ce que** le au moins un tensio-actif est de masse molaire inférieure à 1000.

6. Procédé selon la revendication 4 ou 5, **caractérisé en ce que** le tensio-actif non-ionique présente un indice HLB (Hydrophilic Lipophilic Balance) compris entre 0 et 20.

7. Procédé selon l'une quelconque des revendications précédentes, **caractérisé en ce que** lorsque la composition de vernis est une composition à base aqueuse, l'agent actif est présent dans la phase hydrophobe de l'émulsion simple ou dans la phase hydrophile de l'émulsion multiple.

8. Procédé selon l'une quelconque des revendications 1 à 6, **caractérisé en ce que** lorsque la composition de vernis est une composition à base de solvants à caractère hydrophobe, l'agent actif est présent dans la phase hydrophile de l'émulsion simple ou dans la phase hydrophobe de l'émulsion multiple.

9. Procédé selon l'une quelconque des revendications précédentes, **caractérisé en ce que** l'agent actif est un sel minéral choisi parmi les halogénures, de préférence chlorures ou iodures, les sulfates, de préférence les sulfates d'aluminium, de cuivre, de magnésium, de manganèse, de fer, de potassium, de calcium et de sodium, ou un mélange de ceux-ci.

10. Procédé selon l'une quelconque des revendications précédentes, **caractérisé en ce que** l'agent actif est une molécule organique, ou un mélange de molécules organiques choisie(s) parmi : les vitamines, telles que les vitamines A, B5, C, E, F, H, ou leurs dérivés, des extraits de plantes, de fruits, d'algues, de champignons ou de caviar, des aldéhydes, des huiles végétales, des huiles essentielles, des acides aminés, des peptides, des protéines, des céramides, l'allantoïne et ses dérivés, des dérivés organiques du silicium, des actifs obtenus par fermentation et des dialkylesulfones.

11. Composition de vernis à ongles renfermant un agent filmogène, obtenue par le procédé selon l'une des revendications précédentes dans laquelle au moins un agent actif pour l'ongle est présent dans une phase non miscible avec le solvant dudit vernis et/ou dans une phase distincte de la phase solvant dudit vernis, de manière à être protégé lors du stockage dudit vernis et libéré au contact de l'ongle lors de l'application du vernis sur l'ongle, les proportions pondérales des phases de l'émulsion étant comprises entre 20-80 et 80-20 et ladite émulsion étant présente dans des proportions comprises entre 0,01 % et 1 % en poids de la composition de vernis.

12. Composition de vernis à ongles selon la revendication 11, **caractérisé en ce que** ladite composition étant une composition à base aqueuse, l'agent actif est présent dans une phase hydrophobe ou dans une phase hydrophile en émulsion au sein d'une phase hydrophobe.

13. Composition de vernis à ongles selon la revendication 11, **caractérisé en ce que** lorsque la composition étant une composition à base de solvants à caractère hydrophobe, l'agent actif est présent dans une phase hydrophile ou dans une phase hydrophobe en émulsion au sein d'une phase hydrophile.

14. Composition de vernis à ongles selon la revendication 13, **caractérisé en ce que** la phase hydrophile renferme une teneur en eau inférieure à 0,5 % en poids.

## Patentansprüche

1. Verfahren zum Einarbeiten, in eine Nagellackzusammensetzung mit einem filmbildenden Wirkstoff, eines nagelaktiven Wirkstoffs vom Typ Mineralsalze und/oder organische Verbindung wie Vitamin, Pflanzenextrakt, ätherische Öle zwecks Bewahrung des aktiven Wirkstoffs,
**dadurch gekennzeichnet, dass** es die folgenden aufeinanderfolgenden Schritte umfasst:
a) Herstellen einer Emulsion vom Typ Öl in Wasser oder Wasser in Öl durch Inkontaktversetzen unter Rühren mindestens einer ersten Phase, gebildet aus dem aktiven Wirkstoff oder einer ihn solubilisierenden Lösung, und einer zweiten Phase, in welcher der Wirkstoff unlöslich oder wenig löslich ist, in Anwesenheit mindestens eines Tensids, um eine einfache, stabile Emulsion, gebildet aus zwei Phasen, zu erhalten, wobei die erste diskontinuierliche Phase in Form von Tröpfchen in der zweiten kontinuierlichen Phase vorhanden ist, wobei die zweite Phase in der Lackzusammensetzung löslich ist,
b) dann Einbringen, unter Rühren der Emulsion, in eine Lackzusammensetzung in Verhältnissen zwischen 0,01 und 5 Gew.-% inklusive der Lackzusammensetzung.

2. Verfahren nach Anspruch 1, **dadurch gekennzeichnet, dass** die Emulsion in die Lackzusammensetzung gemäß Verhältnissen zwischen 0,1 und 3 Gew.-% inklusive der Lackzusammensetzung eingebracht wird.

3. Verfahren nach Anspruch 1 oder 2, **dadurch gekennzeichnet, dass** es einen Schritt a)bis umfasst, in welchem die einfache Emulsion, erhalten nach Schritt a) vor dem Schritt b) des Einbringens in die Lackzusammensetzung, unter Rühren in eine dritte Phase eingearbeitet wird, in welcher die zweite Phase der einfachen Emulsion unlöslich ist, um eine multiple, stabile Emulsion zu erhalten, die von drei Phasen gebildet ist, wobei die dritte Phase in der Lackzusammensetzung löslich ist.

4. Verfahren nach einem der vorangehenden Ansprüche, **dadurch gekennzeichnet, dass** das vor dem Inkontaktversetzen der Phasen in mindestens eine der Phasen der Emulsion eingebrachte Tensid oder das Tensidgemisch aus den kationischen, anionischen, amphoteren oder nichtionischen Tensids ausgewählt wird.

5. Verfahren nach einem der vorangehenden Ansprüche, **dadurch gekennzeichnet, dass** das mindestens eine Tensid eine molare Masse unter 1000 hat.

6. Verfahren nach Anspruch 4 oder 5, **dadurch gekennzeichnet, dass** das nichtionische Tensid einen HLB-Index (Hydrophilic Lipophilic Balance) zwischen 0 und 20 inklusive aufweist.

7. Verfahren nach einem der vorangehenden Ansprüche, **dadurch gekennzeichnet, dass**, wenn die Lackzusammensetzung eine Zusammensetzung auf wässriger Basis ist, der aktive Wirkstoff in der hydrophoben Phase der einfachen Emulsion oder in der hydrophilen Phase der multiplen Emulsion vorhanden ist.

8. Verfahren nach einem der Ansprüche 1 bis 6, **dadurch gekennzeichnet, dass**, wenn die Lackzusammensetzung eine Zusammensetzung auf der Basis von Lösungsmitteln mit hydrophobem Charakter ist, der aktive Wirkstoff in der hydrophilen Phase der einfachen Emulsion oder in der hydrophoben Phase der multiplen Emulsion vorhanden ist.

9. Verfahren nach einem der vorangehenden Ansprüche, **dadurch gekennzeichnet, dass** der aktive Wirkstoff ein Mineralsalz ist, ausgewählt aus den Halogeniden, vorzugsweise Chloriden oder Jodiden, den Sulfaten, vorzugsweise den Aluminium-, Kupfer-, Magnesium-, Mangan-, Eisen-, Kalium-, Kalzium- und Natriumsulfaten, oder einem Gemisch derselben.

10. Verfahren nach einem der vorangehenden Ansprüche, **dadurch gekennzeichnet, dass** der aktive Wirkstoff ein organisches Molekül oder ein Gemisch organischer Moleküle ist, das/die ausgewählt ist/sind aus: den Vitaminen wie den Vitaminen A, B5, C, E, F, H oder ihren Derivaten, Extrakten von Pflanzen, Früchten, Algen, Pilzen oder Kaviar, Aldehyden, Pflanzenölen, ätherischen Ölen, Aminosäuren, Peptiden, Proteinen, Ceramiden, dem Allantoin und seinen Derivaten, organischen Siliziumderivaten, Wirkstoffen, erhalten durch Fermentierung und Dialkylsulfonen.

11. Nagellackzusammensetzung mit einem filmbildenden Wirkstoff, erhalten durch das Verfahren nach einem der vorangehenden Ansprüche, in welcher mindestens ein aktiver Wirkstoff für den Nagel in einer mit dem Lösungsmittel des Lacks nicht mischbaren Phase und/oder in einer Phase, die sich von der Lösungsphase des Lacks unterscheidet, derart vorhanden ist, dass er bei der Lagerung des Lacks geschützt ist und im Kontakt mit dem Nagel beim Auftragen des Lacks auf den Nagel freigegeben wird, wobei die Gewichtsverhältnisse der Phasen der Emulsion zwischen 20-80 und 80-20 inklusive sind und die Emulsion in Verhältnissen zwischen 0,01 und 1 Gew.-% inklusive der Lackzusammensetzung vorhanden ist.

12. Nagellackzusammensetzung nach Anspruch 11, **dadurch gekennzeichnet, dass** die Zusammensetzung eine Zusammensetzung auf wässriger Basis ist, wobei der aktive Wirkstoff in einer hydrophoben Phase oder in einer hydrophilen Phase in Emulsion innerhalb einer hydrophoben Phase vorhanden ist.

13. Nagellackzusammensetzung nach Anspruch 11, **dadurch gekennzeichnet, dass**, wenn die Zusammensetzung eine Zusammensetzung auf der Basis von Lösungsmitteln mit hydrophobem Charakter ist, der aktive Wirkstoff in einer hydrophilen Phase oder in einer hydrophoben Phase in Emulsion innerhalb einer hydrophilen Phase vorhanden ist.

14. Nagellackzusammensetzung nach Anspruch 13, **dadurch gekennzeichnet, dass** die hydrophile Phase einen Wassergehalt von unter 1 Gew.-%, vorzugsweise von unter 0,5 Gew.-%, einschließt.

## Claims

1. Method for incorporating, in a nail varnish composition containing a film-forming agent, an active nail agent of mineral salt and/or organic compound type such as a vitamin, plant extract, essential oils with a view to preserving said active agent,
**characterized in that** it comprises the following successive steps:
a) preparing an emulsion of oil-in-water or water-in-oil type by contacting under agitation at least one first phase composed of said active agent, or a solution solubilising the same, with a second phase in which the agent is insoluble or scarcely soluble, in the presence of at least one surfactant to obtain a stable, single emulsion composed of two phases, the first discontinuous phase being present in droplet form within the second continuous phase, said second phase being soluble in the varnish composition;
b) adding said emulsion, under agitation, to a varnish composition in proportions of between 0.01 % and 5 % by weight of the varnish composition.

2. The method according to claim 1, **characterized in that** the emulsion is added to the varnish composition in proportions of between 0.1 and 3 % by weight of said varnish composition.

3. The method according to claim 1 or 2, **characterized in that** it comprises a step a)bis at which the single emulsion obtained after step a), before being added at step b) to the varnish composition, is incorporated under agitation in a third phase in which the second phase of the single emulsion is insoluble, to obtain a stable, multiple emulsion composed of three phases, said third phase being soluble in the varnish composition.

4. The method according to any of the preceding claims, **characterized in that** the surfactant or mixture of surfactants added to at least one of the emulsion phases, before the contacting of said phases, is selected from among cationic, anionic, amphoteric or non-ionic surfactants.

5. The method according to any of the preceding claims, **characterized in that** the at least one surfactant has a molar mass of less than 1000.

6. The method according to claim 4 or 5, **characterized in that** the non-ionic surfactant has a HLB number (Hydrophilic Lipophilic Balance) of between 0 and 20.

7. The method according to any of the preceding claims, **characterized in that** if the varnish composition is an aqueous composition, the active agent is contained in the hydrophobic phase of the single emulsion or in the hydrophilic phase of the multiple emulsion.

8. The method according to any of claims 1 to 6, **characterized in that** if the varnish composition is a solvent composition of hydrophobic type, the active agent is contained in the hydrophilic phase of the single emulsion or the hydrophobic phase of the multiple emulsion.

9. The method according to any of the preceding claims, **characterized in that** the active agent is a mineral salt selected from among halides preferably chlorides or iodides, sulfates preferably aluminum, copper, magnesium, manganese, iron, potassium, calcium and sodium sulfates, or a mixture thereof.

10. The method according to any of the preceding claims, **characterized in that** the active agent is an organic molecule or a mixture of organic molecules selected from among: vitamins, such as vitamins A, B5, C, E, F, H or derivatives thereof, extracts of plants, fruit, algae, fungi or caviar, aldehydes, vegetable oils, essential oils, amino acids, peptides, proteins, ceramides, allantoin and derivatives thereof, organic derivatives of silicone, active substances obtained by fermentation and dialkyl sulfones.

11. Nail varnish composition containing a film-forming agent obtained with the method according to one of the preceding claims, wherein at least one active nail agent is contained in a phase non-miscible with the solvent of said varnish and/or in a phase separate from the solvent phase of said varnish, so that it is protected during storage of said varnish and released on contact with a nail when the varnish is applied to the nail, the weight proportions of the emulsion phases being between 20:80 and 80:20 and said emulsion being contained in proportions of between 0.01 % and 1 % by weight in the varnish composition.

12. The nail varnish composition according to claim 11, **characterized in that** if said composition is an aqueous composition the active agent is contained in a hydrophobic phase or in a hydrophilic phase in emulsion in a hydrophobic phase.

13. The nail varnish composition according to claim 11, **characterized in that** if the composition is a solvent composition of hydrophobic type, the active agent is contained in a hydrophilic phase or in a hydrophobic phase in emulsion in a hydrophilic phase.

14. The nail varnish composition according to claim 13, **characterized in that** the hydrophilic phase has a water content of less than 0.5 % by weight.
